(19) Europäisches Patentamt
European Patent Office
Office européen des brevets

(11) EP 1 439 859 B1

# (12) EUROPEAN PATENT SPECIFICATION

(45) Date of publication and mention of the grant of the patent:
**18.04.2007 Bulletin 2007/16**

(21) Application number: **01993291.2**

(22) Date of filing: **29.10.2001**

(51) Int Cl.:
***A61K 47/48*** *(2006.01)*

(86) International application number:
**PCT/US2001/048568**

(87) International publication number:
**WO 2003/037383 (08.05.2003 Gazette 2003/19)**

## (54) AN ANTINEOPLASTIC- DENDRITIC POLYMER DRUG DELIVERY SYSTEM

ABGABESYSTEM FÜR ANTINEOPLASTISCHE ARZNEISTOFFE AUF BASIS DENDRITISCHER POLYMERE

SYSTEME D'ADMINISTRATION D'UN MEDICAMENT CONTENANT UN DENDRIMERE ANTINEOPLASIQUE

(84) Designated Contracting States:
**AT BE CH CY DE DK ES FI FR GB GR IE IT LI LU MC NL PT SE TR**

(43) Date of publication of application:
**28.07.2004 Bulletin 2004/31**

(73) Proprietor: **Dendritic Nanotechnologies Inc.**
**Mt. Pleasant, MI 48858 (US)**

(72) Inventors:
- **MALIK, Navid**
  **London NW6 6BD (GB)**
- **DUNCAN, Ruth**
  **London NW1 1AX (GB)**
- **TOMALIA, Donald, A.**
  **Midland, MI 48640 (US)**
- **ESFAND, Roseita**
  **Mississauga Ontario LSKIY3 (CA)**

(74) Representative: **Raynor, John et al**
**Beck Greener**
**Fulwood House,**
**12 Fulwood Place,**
**London WC1V 6HR (GB)**

(56) References cited:
**EP-A- 0 928 813** **EP-A- 1 118 333**
**WO-A-01/07469**

- **CHIE KOJIMA, KENJI KONO, KAZUO MARUYAMA, AND TORU TAKGISHI: "Synthesis of Polyamidoamine Dendrimers Having Poly (ethylene glycol) Grafts and Their Ability To Encapsulate Anticancer Drugs" BIOCONJUGATE CHEMISTRY, vol. 11, no. 6, November 2000 (2000-11), pages 910-917, XP002201126**
- **UHRICH K: "Hyperbranched Polymers for Drug Delivery" TRENDS IN POLYMER SCIENCE, ELSEVIER SCIENCE PUBLISHERS B.V. AMSTERDAM, NL, vol. 5, no. 12, 1 December 1997 (1997-12-01), pages 388-393, XP004098786 ISSN: 0966-4793**
- **JANSEN J F G A ET AL: "ENCAPSULATION OF GUEST MOLECULES INTO A DENDRITIC BOX" SCIENCE, AMERICAN ASSOCIATION FOR THE ADVANCEMENT OF SCIENCE,, US, vol. 266, no. 5188, 18 November 1994 (1994-11-18), pages 1226-1229, XP000500113 ISSN: 0036-8075**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 439 859 B1

## Description

This invention relates to the treatment of cancer in animals, especially humans, using dendritic polymer conjugates having an antineoplastic drug present.

The use of polymers as carriers for drugs, especially those drugs that have low water solubility at physiological pH, are toxic to the normal tissue, or cannot be administered in sufficient dosage, has gained interest in recent years [*e.g.,* H. Ringsdorf, *J. Polymer Sci.*: Symp. 51, 135-153 (1975)]. A polymer carrier for antineoplastic drugs would provide a useful system for administration of these drugs because of their solubility, toxic and higher dose at delivery characteristics. Several efforts to deliver doxorubicin are illustrative of this effort [*e.g.,* R. Duncan *et al.,* "Preclinical Toxicology of a Novel Polymeric Antitumor Agent: I-copolymer-doxorubicin (PK1)", *Hum. Exp. Toxiocol.* 17(2), 93-104 (1998); P. A. Vassey *et al.,* "Phase I Clinical and Pharmacokinetic Study of PK1 [N-(2-Hydroxypropyl)methacrylamide Copolymer Doxorubicin]: First Member of a New Class of Chemotherapeutic Agents - Drug-Polymer Conjugates", *Clin. Cancer Res.* 5, 83-94 (1999); L. W. Seymour *et al.*, "N-(2-Hydroxypropyl)methacrylamide Copolymers Targeted to the Hepatocyte Galactose-receptor;Pharmacokinetics in $DBA_2$ Mice", *Br. J. Cancer* 63, 859-866 (1991).

The prospect of using dendritic polymers as caters or carriers for drug delivery has been previously proposed on account of the unique structure and characteristics of these polymer molecules [R. Esfand and D. A. Tomalia, "Poly (amidoamine) (PAMAM) Dendrimers: from Biomimicry to Drug Delivery and Biomedical Applications", research focus, DDT 6(8), 427-436 (8 April 2001); US Patents 5,338,532 and 5,527,524 Kojima et al., Bioconjugate Chemistry 11(6): 910-917 (2000), WO 01/07469; Uhrich, K., Trends in Polymer Science 5(12): 388-393 (1997)]. More specifically, it has been proposed that the external surface functionality and interior morphological characteristics of dendritic polymer molecules appear to be very promising for developing new methods for controlling drug release and targeted drug delivery systems. However, relatively little work has been done in specific areas of drug delivery. In particular, the use of dendritic polymers as effective caters for specific anti-tumor agents has not heretofore been demonstrated.

Certain platinum containing compounds, particularly carboplatin (*cis*-diamine(1,1-cylobutanedicarboxylato) platinum (II)) and cisplatin (*cis*-diamminedichloroplatinum), have been used in the treatment of ovarian cancer, lung cancer, testicular cancer, breast cancer, stomach cancer and lymphoma. However, because of the non-specific toxicity and poor water solubility of these platinum-containing compounds, the use of carboplatin and cisplatin has been relatively limited.

In order to overcome the non-specific toxicity and water solubility problems associated with cisplatin and carboplatin, it has been proposed to use linear polymers as carriers for these drugs. However, the use of linear polymers as caters in drug delivery systems has several disadvantages. A major disadvantage with linear polymer drug carriers is that they are heterogeneous, polydisperse compositions containing various different molecular weight polymer molecules with a limited number of functional groups and/or reactive sites. Because linear polymer compositions are not comprised of molecules having a precisely defined structure, it is more difficult to maintain uniform polymer properties, drug delivery properties, and therapeutic efficacy. As a result it is relatively difficult to obtain governmental regulatory approval of the linear polymer-drug composites. Another disadvantage with the use of linear polymers as drug-carriers is that the location, and hence the availability, of the drug is difficult to control. In particular, the drug must either be bound covalently or non-covalently in a random unpredictable manner and the linear polymer structure lacks well-defined cargo space for the drug. The tendency of the drug to become buried in the linear polymer leads to greater unpredictability on account of the non-uniform or heterogeneous properties of the linear polymer molecules, and results in reduced drug efficiency because a significant proportion of the drug molecules are not effectively presented to the cell being treated. In some cases the random coil structure of the linear polymers may even prevent successful drug attachment within the coil and lead to passive entrapment, leading to uncontrolled drug release (*e.g.,* random diffuse system), *i.e.,* lack of uniformity in the timing of the drug release.

Accordingly, it would be highly desirable to provide a precisely defined drug delivery system for cisplatin and carboplatin as well as related antineoplastic agents which exhibit high drug efficiency, high drug carrying capacity, good water solubility, good stability on storage, reduced toxicity, and improved anti-tumor activity *in vivo.*

US Patent 5,338,532 teaches polymer conjugates comprising dense polymers associated with a carried material. [One type of dense star polymers is Starburst® polymers (trademark of The Dow Chemical Company) where the dendrimer is a polyamidoamine (PAMAM).] A variety of suitable applications for such conjugates are broadly discussed in US Patent 5,332,532, including the use of these conjugates as delivery vehicles for biologically active agents. However, US Patent 5,338,532 does not specifically teach, claim, or even mention the use of polymer conjugates as delivery vehicles for cisplatin, carboplatin, titanocene dichloride and diorganotin dihalides or other antineoplastic agents. US Patent 5,338,532 only exemplifies the use of zero valence metals, and ionic or radioactive metals, specifically exemplifying Fe, Rh, Pd, Y, Fn, Pb, Gd, Mn and Gd.

A recent publication has disclosed the use of dendrimers as carriers for the delivery of cisplatin, *i.e.,* "Dendrimer-platinate: a Novel Approach to Cancer Chemotherapy", *AntiCancer Drugs,* 10, 767-776 (1999). This publication deals specifically with the formation of a dendrimer-cisplatin conjugate, *i.e.,* a dendrimer-platinate. According to this publication

the dendrimer-cisplatin conjugate is formed by a covalent or ionic interaction between the surface of the dendrimer and the platinum of the cisplatin, releasing a chloride ion in the process. This method of association differs from that of the present invention which involves the conjugation of the dendritic polymer with an antineoplastic agent, (*e.g.*, cisplatin, carboplatin, and other metal-containing analogues thereof) through the encapsulation of the antineoplastic agent within the interior of the dendritic polymer. The method of the present invention is advantageous over that of this publication because the encapsulated conjugate of the present invention provides a method of coupling a higher delivery dose of an antineoplastic agent of a broader class than cisplatin together with a lowering of toxicity.

This invention pertains to dendritic polymer conjugates which are useful drug delivery systems for carrying cisplatin, carboplatin, oxalipatin, teraplatin, platinium-DACH, ormaplatin, titanocene dichloride, vanadocene dichloride, niobocene dichloride, molybdenocene dichloride, rhenocene dichloride, and diorganotin dihalides or other metal containing antineoplastic agents (hereinafter "antineoplastic dendritic polymer conjugates"); preferably cisplatin and carboplatin and other platin-based analogues (hereafter "platin-based analogue dendritic polymer conjugates"); more preferably cisplatin (hereafter "cisplatin dendritic polymer conjugates"), as antineoplastic agents to malignant tumor as antitumor agents. The invention also pertains to methods of treating malignant tumors using these antineoplastic dendritic polymer conjugates, and to a method of preparing an antineoplastic dendritic polymer conjugate useful for carrying platinum (Pt), titanium (Ti), tin (Sn), vanadium (V), niobium (Nb), molybdenum (Mo), or rhenium (Re) containing compounds such as those agents named above (collectively "antineoplastic agents") to malignant tumors.

The antineoplastic dendritic polymer conjugates comprise a dendritic polymer conjugated to an antineoplastic agent, forming a dendritic polymer antineoplastic conjugate, *e.g.*, especially a platin-based dendritic polymer conjugate. These antineoplastics dendritic polymer conjugates are prepared by obtaining a dendritic polymer having functional groups or chelational groups which are accessible to a antineoplastic agent and capable of interacting with the functional or chelational groups (chelated through a linker to the surface of the dendrimer or preferably in the interior of the dendrimer - "cargo space"), contacting the dendritic polymer with the antineoplastic agent, and thereby allowing for uptake and encapsulation of the antineoplastic agent by the dendritic polymer by means of covalent and/or non-covalent interactions, *e.g.*, physically encapsulated within the interior of the dendrimer, dispersed partially or fully throughout the dendrimer, or linked to the dendrimer with a chelation group, or any combination thereof, whereby the attachment or linkage is by means of covalent bonding, hydrogen bonding, adsorption, adsorption, metallic bonding, dipole-dipole interaction, van der Waals forces, or ionic bonding, or any combination thereof. These antineoplastic dendritic polymer conjugates are administered to an animal having a malignant tumor in an amount which is effective to inhibit growth of the malignant tumor, preferably intravenously (I.V.), although other methods such as oral, parental I.P.), subcutaneous (S.C.), intramuscular, intraarterial or topical administration are also possible.

The antineoplastic dendritic polymer conjugate results in an anti-tumor agent that exhibits unexpected and surprisingly high efficacy, drug carrying capacity, and dosage capabilities. The antineoplastic dendritic polymer conjugate also shows a surprising and unexpected decrease in toxicity, good water solubility, good stability on storage, and improved anti-tumor activity *in vivo.* Most significantly, these antineoplastic dendritic polymer conjugates were found to be active against B16F10 tumor models, which are known to be resistant to cisplatin at its maximum tolerated dose via I.V. administration (about 1 mg/kg).

FIG. 1 is a graph showing the effect of cationic dendrimers on hemolysis of rat erythrocytes at 1 hour;
FIG. 2 is a graph showing the effect of anionic dendrimers on hemolysis of rat erythrocytes at 1 hour;
FIG. 3 is a graph showing the effect of anionic dendrimers on BI6F10 cells at 72 hours;
FIG. 4 is a graph showing the effect of cationic dendrimers on B16F10 cells at 72 hours;
FIG. 5 is a graph showing the effect of cationic dendrimers on CCRF-CEM cells at 72 hours;
FIG. 6 is a graph showing the effect of anionic dendrimers on CCRF-CEM cells at 72 hours;
FIG. 7 is a graph showing the effect of anionic dendrimers on HepG2 cells at 72 hours;
FIG. 8 is a graph showing the effect of cationic dendrimers on HepG2 cells at 72 hours;
FIG. 11 is a graph showing the release of cisplatin from a dendrimer-platinate at two physiological pH conditions at 72 hours and 37°C;
FIG. 10 is a bar graph showing the effect of intraperitoneal injection of dendrimer-platinum conjugate treatment on intraperitoneally injected tumors;
FIG. 11 is a bar graph showing the effect of dendrimer-platinum conjugate on established B16 melanoma;

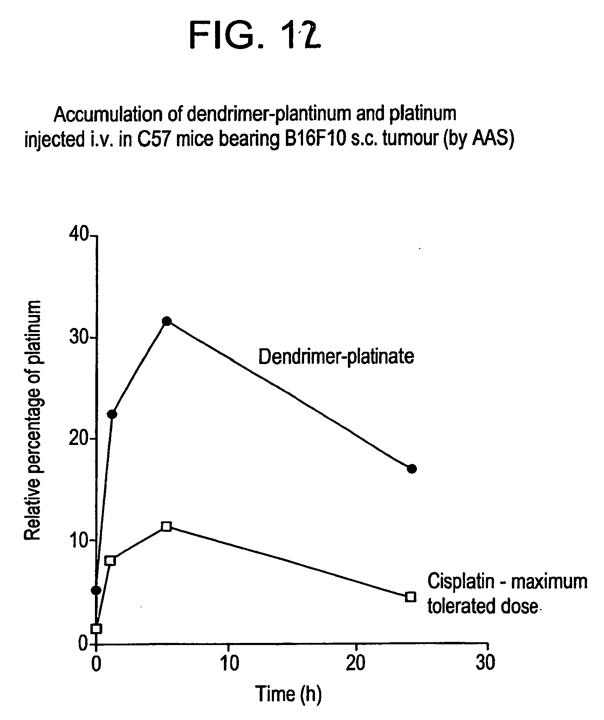

FIG. 12 is a graph showing the accumulation of dendrimer-platinum and platinum intravenously injected in C57 mice bearing B16FI0 subcutaneously implanted tumor;

The dendritic polymers which may be used to form antineoplastic dendrimer polymer conjugates include generally any of the known dendritic architectures including dendrimers, controlled hyperbranched polymers, dendrigrafts, and random hyperbranched polymers. Dendritic polymers are polymers with densely branched structures having a large number of reactive groups. A dendritic polymer includes several layers or generations of repeating units which all contain

one or more branch points. Dendritic polymers, including dendrimers and hyperbranched polymers, are prepared by condensation reactions of monomeric units having at least two reactive groups after attachment. The dendrimers that can be used include those comprised of a plurality of dendrons that emanate from a common core which can be a single atom or a group of atoms. Each dendron generally consists of terminal surface groups, interior branch junctures having branching functionalities greater than or equal to two, and divalent connectors that covalently connect neighboring branching junctures. For a review article of this area see, for example, Donald A. Tomalia, *et al., Angew. Chem. Int. Engl.* 29, 138-175 (1990).

Dendrons and dendrimers can be prepared by convergent or divergent synthesis.

Divergent synthesis of dendrons and dendrimers involves a molecular growth process which occurs through a consecutive series of geometrically progressive step-wise additions of branches upon branches in a radially outward molecular direction to produce an ordered arrangement of layered branched cells. Each dendritic macromolecule includes a core branch cell, one or more layers of internal branch cells, and an outer layer of surface branch cells, wherein each of the cells includes a single branch juncture. The cells can be the same or different in chemical structure and branching functionality. The surface branch cells may contain either chemically reactive or passive functional groups. Chemically reactive surface groups can be used for further extension of dendritic growth or for modification of dendritic molecular surfaces. The chemically passive groups may be used to physically modify dendritic interiors or dendritic surfaces, such as to adjust the ratio of hydrophobic to hydrophilic terminals. In this fashion one can improve the solubility of a guest molecule in the interior of the dendritic polymer or the solubilization of the dendritic container in a particular solvent. (See for example for dense star polymers US Patents 4,507,466; 4,588,120; 4,568,737; 4,631,337; 4,587,329; and 4,737,550; WO 84/02705; EP 0115771; and EP 0608908; for rod shaped dense star polymers US Patent 4,694,064; EP 02344008; and EP 0556871; for hydrophobic outer shell dense star polymers US Patent 5,560,929; and EP 0680495.)

Convergent synthesis of dendrimers and dendrons involves a growth process, which begins from what will become the surface of the dendron or dendrimer, and progresses radially in a molecular direction toward a focal point or core. (See for example US Patent 5,041,516.) The dendritic polymers may be ideal or non-ideal, i. e., imperfect or defective. Imperfections are normally a consequence of either incomplete chemical reactions, or unavoidable competing side reactions. In practice, real dendritic polymers are generally nonideal, *i.e.,* contain certain amounts of structural imperfections.

The hyperbranched polymers which may be used represent a class of dendritic polymers which contain high levels of nonideal, irregular branching as compared with the more nearly perfect regular structure of dendrons and dendrimers. Specifically, hyperbranched polymers contain a relatively high number of irregular branching areas in which not every repeat unit contains a branch juncture.

The preparation and characterization of dendrimers, dendrons, random hyperbranched polymers, controlled hyperbranched polymers, and dendrigrafts (collectively "dendritic polymers") is well known. Examples of dendrimers and dendrons, and methods of synthesizing the same are set forth in US Patents 4,410,688, 4,507,466; 4,558,120; 4,568,737; 4,587,329; 4,631,337; 4,694,064; 4,713,975; 4,737,550; 4,871,779 and 4,857,599. Examples of hyperbranched polymers and methods of preparing the same are set forth, for example in US Patents 5,418,301 and 5,514,764. Examples of dendrigrafts and methods of preparing the same are set forth, for example in an article by D. A. Tomalia and R. Esfand, *Chem.* & *Ind.,* 416-420 (2 June 1997).

The dendritic polymers or macromolecules useful in the practice of this invention are characterized by a relatively high degree of branching, which is defined as the number average fraction of branching groups per molecule, i. e., the ratio of terminal groups plus branch groups to the total number of terminal groups, branched groups and liner groups. For ideal dendrons and dendrimers, the degree of branching is 1; whereas for linear polymers, the degree of branching is 0. Hyperbranched polymers have a degree of branching that is intermediate to that of linear polymers and ideal dendrimers, preferably of at least about 0.5 or higher. The degree of branching is expressed as follows:

$$f_{br} = \frac{N_t + N_b}{N_t + N_b + N_1}$$

where $N_x$ is the number of type x units in the structure. Both terminal (type t) and branched (type b) units contribute to the fully branched structure whilst linear (type 1) units reduce the branching factor; hence

$$O \leq f_{br} \leq 1$$

where $f_{br}$ = O represents the case of a linear polymer and $f_{br}$ = 1 represents the case of a fully branched macromolecule.

Dendritic polymers also include macromolecules commonly referred to as cascade molecules [*e.g.,* E. Buhleier *et al., Synthesis* 155-158 (Feb. 1978)], arborols [*e.g.,* US Patents 5,376,690 and 5,210,309], arborescent grafted molecules, tectodendrimers [*e.g.,* Srinivas Uppuluri *et al.,* "Tecto(dendrimer) Core-shell Molecules: Macromolecular Tectonics for the Systematic Synthesis of Larger Controlled Structure Molecules" PMSE, Spring Meeting (March 21-25, 1999) 55-56], and the like. Suitable dendritic polymers also include bridged dendritic polymers, *i.e.*, dendritic macromolecules linked together either through surface functional groups or through a linking molecule connecting surface functional groups together, and dendritic polymer aggregates held together by physical forces. Also included are spherical-shaped dendritic polymers (*e.g.*, US Patents 4,507,466; 4,588,120; 4,568,737; 4,631,337; 4,587,329; and 4,737,550) and rod-shaped dendritic polymers (*e.g.*, US Patent 4,694,064) grown from a polymeric core. Additional dendritic polymers suitable for use with the present invention include all the basic dendritic structures where specific chelating groups or moieties are either in the central core of the dendrimer, and/or located within the interior structure on the dendron arms and/or located on the surface of the dendrimer. All of these above dendrimer terms are to be understood to be included within the term "dendritic polymers".

The dendritic polymers can be generationally monodisperse or generationally polydisperse. Dendritic polymers in a monodisperse solution are substantially all of the same generation, and hence of uniform size and shape. The dendritic polymers in a polydisperse solution comprise a distribution of different generation polymers. The dendritic polymer molecules which may be used include mixtures of different interior and exterior compositions or functionalities. Examples of suitable dendritic polymers include poly(ether) dendrons, dendrimers and hyperbranched polymers, poly (ester) dendrons, dendrimers and hyperbranched polymers, poly(thioether) dendrons, dendrimers and hyperbranched polymers, poly(amino acid) dendrons dendrimers and hyperbranched polymers, poly(arylalkylene ether) dendritic polymers and poly(propyleneimine) dendrons, dendrimers and hyperbranched polymers. Poly(amidoamine) (PAMAM) dendrimers have been found to be particularly useful for preparing the metal-containing antineoplastic dendritic polymer conjugates of this invention.

Dendritic polymers which are useful include those that have symmetrical branch cells (arms of equal length, *e.g.*, PAMAM dendrimers; for example described in US Patent 5,527,524) and those having unsymmetrical branch cells (arms of unequal length, *e.g.* lysine-branched dendrimers; for example described in US Patent 4,410,688),) branched dendrimers, cascade molecules [*e.g.*, E. Buhleier *et al., Synthesis* 155-158 (Feb. 1978)], arborols [*e.g.,* US Patents 5,376,690 and 5,210,309], and the like.

*The term "dendritic polymer" also includes so-called "hyper comb-branched" polymers. These comprise non-crosslinked poly-branched polymers prepared by (1) forming a first set of linear polymer branches by initiating the polymerization of a first set of monomers which are either protected against or non-reactive to branching and grafting, during polymerization, each of the branches having a reactive end unit upon completion of polymerization, the reactive end units being incapable of reacting with each other; (2) grafting the branches to a core molecule or core polymer having a plurality of reactive sites capable of reacting, with the reactive end groups on the branches; (3) either deprotecting or activating a plurality of monomeric units on each of the branches to create reactive sites; (4) separately forming a second set of linear polymer branches by repeating step (1) with a second set of monomers; (5) attaching the second set of branches to the first set of branches by reacting the reactive end groups of the second set of branches with the reactive sites on the first set of branches, and then repeating steps (3), (4) and (5) above to add one or more subsequent sets of branches. Such hyper comb-branched polymers are disclosed in European Patent Publication 0473088A2 which are generally referred to as "dendrigraft polymers". A representative formula for such hyper comb-branched polymer is:

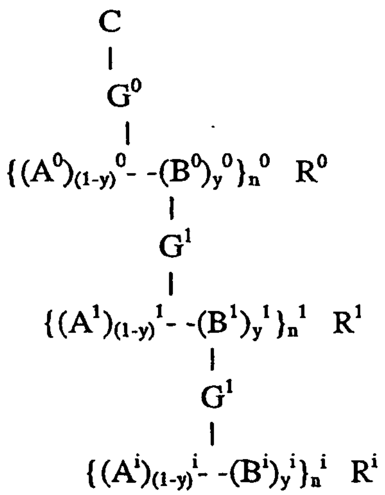

wherein C is a core molecule; each R is the residual moiety of an initiator selected from a group consisting of free radical initiators, cationic initiators, anionic initiators, coordination polymerization initiators and group transfer initiators; A and B are polymerizable monomers or comonomers capable of withstanding the conditions required for branching therefrom or grafting thereto, at least during the polymerization of the {(A)-(B)} linear polymer chain and during its grafting to a prior {(A)-(B)} branch of the {(A)-(B)} core branch; each G is a grafting component and the designation

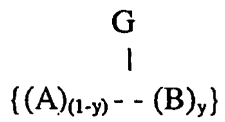

indicates that G can attach to either an (A) unit or a (B) unit; n is the degree of polymerization of the indicated generation comb-branches; y is the fraction of B units in the indicated generation branch, and has a value of .01 to 1; the superscripts 0, 1 and i designate the comb-branch generation level, with i beginning at "2" and continuing for the number of reiterative branch set generations in the polymer; and at least $n^0$ and n' are ≥ 2.

For purposes of clarifying terminology, it should be noted that dense star dendrimers are built by reiterative terminal branching, while hyper comb-branched dendrimers are built by reiterative comb-branching. In dense star dendrimers, subsequent generation branches are attached to the terminal moieties of a previous generation, thus limiting the degree of branching to the functionality of the previous generation terminal moiety, which would typically be two or three. In contrast, by branching oligomers upon prior generation oligomer branches in accordance with hyper comb-branched dendrimer, one can dramatically increase the degree of branching from generation to generation, and indeed can vary the degree of branching from generation to generation.

The dendritic polymers which are believed to be most useful are approximately monodispersed. That is, dendritic polymers in a monodispersed solution in which all of the dendritic polymer molecules are substantially of the same generation, and hence of uniform size and shape, are preferred. Monodispersed solutions of dendrimers are particularly preferred.

The dendritic polymers used have internal and/or terminal functional or chelational groups which are accessible to an antineoplastic agent which is capable of associating with the functional or chelational groups, thereby allowing for the uptake of the antineoplastic agent by the dendritic polymer. Dendritic polymers having anionic terminal functional groups are preferred. Examples of anionic terminal function groups include sulfonates, sulfates and carboxylate groups, with carboxylate or carboxylic groups, including the sodium and potassium salts thereof, being particularly preferred. While not wishing to be bound by theory, it is believed that the advantageous results are obtained because the platin form of the Pt permits it to be active, usually in its hydrated form, in the interior of the dendrimer, and at the cancer cell site it is unloaded or released from the dendrimer in its active form, still believed to be the hydrated form, and then the Pt is able to cross-link with the DNA of the cancer cell and thereby inhibit the proliferation of the cancer cells. When the EPR antineoplastic dendritic polymer conjugate is injected upstream of the tumor mass it will enter easily as it size is controlled by the dendrimer and the tumor has a large incoming blood flow and it will not leak to the surrounding area as the vascular size leaving the tumor is more restricted and the dendrimer size too large for those vessels. Thus a high

concentration of the drug is kept in the tumor. [See for example UM. Ohndorf *et al., Nature* 399, 708-712 (17 June 1999).] While not wishing to be bound by theory, it is believed that the advantageous results are obtained because the antineoplastic agents are positively charged and are attracted to the negative charge of the carboxylic groups on the dendrimer and with a higher osmotic pressure on the outside of the dendrimer than in its interior a shunting of the antineoplastic agent occurs to move it into the interior of the dendrimer.

Encapsulation or entrapment is a chemical or physical interaction based on ionic or any other form of association between two compounds (*e.g.*, covalent bonding, hydrogen bonding, adsorption, absorption, metallic bonding, van der Waals forces between a host molecule (dendritic polymer) and a guest molecule (an antineoplastic agent). Encapsulation can be reversible or irreversible. The dendrimer and antineoplastic agent encapsulation of the present invention defines the dendrimer to act as a host, port, or site for the antineoplastic agent, *i.e.,* especially cisplatin or carboplatin. In the area of polymer chemistry because dendrimers have specific structural related properties, encapsulation is a more defined and accurate term. In contrast, with linear polymers where it is not really clear where the drug is bound or associated to the polymer, the term entrapment is more commonly used.

It is also possible to covalently attach an antineoplastic agent to the dendrimer. This covalent attachment may be directly between the interior surface of the dendrimer and the antineoplastic agent or by means of a linker moiety between the surface of the dendrimer and the antineoplastic agent. Some linkers that may be used are described in US Patent 5,527,524; EP 0353450; EP 0570575; and EP 0296522.

Any surface association between the antineoplastic agent and the dendritic polymer forms via a covalent or ionic interaction, thereby allowing for the antineoplastic agent to be associated with the dendritic polymer surface long enough for a second interaction to occur between the interior amine groups of the dendrimer and the antineoplastic agent. Thus, resulting in an encapsulated conjugate by a charged shunt mechanism, resulting in the encapsulation or uptake of the antineoplastic agent by the dendritic polymer. Thus, dendritic polymers having anionic terminal functional groups are preferred. Examples of anionic terminal function groups include sulfonates, sulfates and carboxylate groups, with carboxylate or carboxylic groups and their salts being particularly preferred.

Examples of suitable dendritic polymers which may be used include poly(amidoamine) dendrimers, especially carboxylate terminated poly(amidoamine) dendrimers, and carboxylate terminated poly(propyleneimine) dendrimers, especially where these carboxylic acid groups have formed salts, especially sodium or potassium.

The generation of the dendritic polymer, and hence the size of the dendritic polymer, which may be utilized may vary considerably. For example, generation 3.5 poly(anudoamine) dendrimers (3.5 PAMAM) are acceptable for this use. However, higher and lower generations are also expected to be useful, but especially the range from generation 3.5 to 7.5 for PAMAM dendrimers, having an ethylenediamine (EDA) core.

The antineoplastic agent can be generally any antineoplastic agent, especially a platin-based analogue, which can be reversibly encapsulated within the dendritic polymer or chelated with a linker to the dendritic polymer surface and which exhibits anti-tumor activity when released from the dendritic polymer. An antineoplastic agent is a therapeutic compound used for the treatment of neoplastic diseases, such as ovarian cancer, lung cancer, testicular cancer, breast cancer, stomach cancer and lymphoma. Examples of antineoplastic agent, as used herein, are as defined before. The preferred antineoplastic agent is a platinum containing compound, *e.g.,* a platin-based analogue. More preferred are cisplatin (cis-diamminedichloroplatinum) [see B. Rosenberg *et al., Nature* 205, 698 (1965), German Patents 2,318,020 and 2,329,485] and carboplatin (cis-diammine(1,1-cyclobutane-dicarboxylato)platinum) [see US Patent 4,140,707]. Other suitable platinum containing compounds include those having a tetravalent platinum atom bonded to the nitrogen of two amine ligands, which may be the same or different, the amine ligands being in *cis* conformation with respect to each other, the remaining ligands may be capable of interacting with or being displaced by a functional group of the dendritic polymer. An example of such a compound is *cis*-diamminedichloroplatinum. A large number of different analogues of cisplatin have been investigated [see for example; J. Respondek and J. Engel, *Drugs Of The Future* 21(4), 391-408 (1996) and R.B. Weiss and M.C. Christian, *Drugs* 46, 360-377 (1993)] and many of these different platinum-derivatives are likely to be useful in the present invention and are included within the term "platin-based analogues".

Antineoplastic dendritic polymer conjugates may be prepared by dissolving a dendritic polymer in a suitable solvent, such as water, contacting the dissolved dendritic polymer with a dissolved antineoplastic agent under conditions sufficient to cause the antineoplastic agent to associate with the dendritic polymer and to form a dendritic polymer-antineoplastic conjugate. A cisplatin to dendrimer (Generation 3.5 PAMAM, EDA core, dendrimer) molar ratio of 35:1 was used in many of the experiments described in the examples. The ratio of cisplatin molecules to dendritic polymer molecules can vary considerably. Dendritic polymer platmates having a cisplatin to dendritic polymer molar ratio of from about 100:1 to about 1:1 have been evaluated and are expected to provide practical advantages. The antineoplastic agents used are encapsulated within the dendrimer which it is believed to be by using a shunt mechanism whereby the anionic groups (*e.g*., carboxylate groups) of the dendrimer surface are responsible for a weak dendrimer-antineoplastic agent interaction that allows for the antineoplastic agent to be uptaken by the dendrimer (*i.e.,* primary interaction), possibly proceeding through a reaction between the interior nitrogen groups of the dendrimer and the antineoplastic agent (*i.e.*, secondary interaction). This ionic shunt mechanism results in the encapsulation of the antineoplastic agent

within the dendritic polymer. In addition to encapsulation it is also believed that some surface association of the antineoplastic agent with the dendritic polymer may occur.

The antineoplastic dendritic polymer conjugate may be administered to animals, especially humans, in a therapeutically effective amount to treat a malignant tumor in the animal. The antineoplastic dendritic polymer may be administered orally or topically, but are preferably administered parentally, such as by subcutaneous (S.C.) injection, intraperitoneal (I.P.) injection, intravenous (I.V.) injection, intraaterial injection or intramuscular injection. An effective amount of a generation 3.5 poly(amidoamine) dendrimer-cisplatin conjugate in which the cisplatin loading is about 25% by weight (*i.e.*, 25% by weight of the conjugate is cisplatin) has been found to be from about 1 milligram per kilogram (1 mg/kg) of body weight to about 15 milligrams per kilogram (15 mg/kg) of body weight for a mouse (DBA2 or C57) for an intraperitoneal injection. Suitable quantities of various antineoplastic dendritic polymer conjugates which are therapeutically effective in the treatment of various malignant tumors in other animals can be determined through routine experimentation and testing.

It is anticipated that the antineoplastic dendritic polymer conjugates will be effective in the treatment of various malignancies in which cisplatin, carboplatin and other antineoplastic agents as anti-tumor agents have been found to be therapeutically affective, including ovarian cancer, lung cancer, testicular cancer, breast cancer, stomach cancer and lymphoma. Also it is anticipated that the antineoplastic dendritic polymer conjugates could be used in combination therapy with other anticancer agents (*i.e.,* synergistic application). *In vitro* testing and *in vivo* testing on mice suggest that the antineoplastic dendritic polymer conjugates, especially the platin-based analogues, are also therapeutically effective in the treatment of melanoma and human lymphoblastic leukemia.

Glossary of terms in the Examples:

AAS means atomic absorption spectroscopy
AUC means area under the curve
BDH means BDH Laboratory Supplies in Dorest, England
cisplatin means *cis*-diamminedichloro platinate (II)
carboplatin means *cis*-diammine(1,1-cyclobutanedicarboxylato)platinate (II)
DDW means double deionized water
DMSO means dimethylsulfoxide
EDA means ethylenediamine
EPR means enhanced permeability retention
FCS means fetal calf serum
GPC means gel permeation chromatography
I.P. means intraperitoneal
IR means infrared spectroscopy
I.V. means intravenous
MEM means minimal essential media
MTT means 3-(4,5-dimethyltiazol-2-yl)-2,5-diphenyl tetrazolium bromide (a colorimetric dye which is a pale yellow substrate that is cleaved by living cells to yield a dark blue formazan product)
MWCO means molecular weight cut off
NMR means nuclear magnetic resonance spectroscopy
OD means optical density
OPDA means o-phenylenediamine assay (added to perform photometry assay for metals)
PAMAM means poly(amidoamine) dendrimers
PBS means phosphate buffered saline
POPAMS means poly(propyleneimine) dendrimers
PSC means particle sizing by photon correlation spectroscopy
RPMI media means Roswell Park Memorial Institute media, usually RPMI -1640, see G. E. Moore and L. K. Woods, "Culture Media for Human Cells - RPMI 1603" RPMI 1634, RPMI 1640 and RPMI GEM 1717", *Tissue Culture Assoc. Manual* 3, 503-508 (1976)
S.C. means subcutaneous

Experimental Methods

Synthesis and Characterization

Poly(amidoamine) dendrimers (PAMAM, EDA core) (Sigma) were synthesized according to the method of Tomalia *et al., Polymer J.,* 17, 117-132(4) (1985). Dendrimers of generation 3.5 (COONa) and 4 ($NH_2$) were allowed to interact with cisplatin under stirring conditions at room temperature for 4 hours.

| Dendrimer Generation | MW (Daltons) | No. Funct. Groups |
|---|---|---|
| 4.0 | 14,215 | 64 ($NH_2$) |
| 3.5 | 12,419 | 64 (COONa) |

The dendrimer-platinum (Pt) was characterized using the OPDA (colorimetric) assay and AAS (total Pt), GPC (Mw and free Pt), IR and NMR.

Pt Release

To study the rate of Pt release and also dendrimer biodegradation the conjugate was incubated in buffers at pH 7.4 and 5.5 and also in the presence of serum and lysosomal enzymes.

Biological Evaluation

*In vitro* cytotoxicity was assessed against B16F10 melanoma, CCRF (human lymphoblastic leukemia) and Cor-L23 (human lung) cells using the MTT assay. Dendrimer-Pt and free cisplatin were administered I.P. (days 1,2,3 or day 1 only) to DBA2 or C57 mice bearing I.P. inoculated L1210 or B16F10 tumors (respectively). Alternatively drug was administered I.V. to mice bearing S.C. implanted B16F10 when the tumor reached palpable size (50-100 $mm^2$). Animal weight, tumor size and animal survival were monitored (UK guidelines for animal experiments involving neoplasia were followed.)

Materials

Polyamidoamine (PAMAM, EDA core) Starburst® dendrimers (trademark of The Dow Chemical Company) were purchased from Aldrich (UK) Ltd.

The following examples further illustrate the invention but are not considered as a limitation on the scope of the invention.

EXAMPLES

Example 1 Effect of PAMAM dendrimer on the stability of rat erythrocytes incubated *in vitro.*

Method

Poly(amidoamine) dendrimers (cationic and anionic) of increasing generations were incubated with rat erythrocytes obtained from an adult Wistar rat. The interaction of the dendrimer with the erythrocyte was assessed spectrophotometrically by the detection of released hemoglobin, induced by lysis, with a spectrophotometer at 550 nm. Various concentrations of dendrimer, controls (methanol (BDH)), poly-L-lysine (HBr salt - 56.5 KD Mw (Sigma)), and dextran (74 KD Mw (Sigma)) (dissolved in physiologically buffered saline) were incubated with the rat erythrocytes (2% w/v solution) for 1 hour at 37°C, and at 10 rpm (shaking water bath). On completion, the erythrocytes were spun in a centrifuge at 1500 x g for 10 minutes to pellet the cells and 100 μl of the supernatant was removed and analyzed on the spectrophotometer after blanking against PBS. The results are expressed in FIGs. 1 and 2 as a percentage of hemoglobin release compared to an intrinsic control (Triton x 100 (1% v/v solution (Sigma)) which gave 100% lysis.

Result

Cationic dendrimers, except generation 1, were lytic, whereas soluble anionic dendrimers (including PAMAM generation 3.5) were not lytic.

Example 2 Cell Cytotoxicity of unmodified dendrimers against B 16F10 cells

Method

B 16F10 cells are an adherent murine melanoma cell line. B 16F10 cells were seeded at a density of 1 x $10^5$ cells per ml (1 x $10^4$ cells per well) in a 96 well flat bottomed microtitre plate (Costar) in RPMI 1640 tissue culture media (Gibco) supplemented with 10% FCS (Gibco). All cellular growth and cytotoxic incubations were carried out in a cell incubator at 37°C and 5% $CO_2$ atmosphere.

Cell density was assessed using an improved neurenbrow hemocytometer (Sigma). The cells were washed with PBS twice and fresh RPMI media (supplemented with FCS) was added, and the cells were then seeded in a microtitre plate. The cells were left for 24 hours to recover and re-adhere.

All polymers and controls were dissolved in RPMI media (supplemented with FCS) and then sterilized through a 0.2 μm sterile filter (Acrodisk), the first few microliters of the solution being discarded in the case of adherence of the polymer to the filter membrane. Polymer and controls were then added in increasing concentrations to the cells in the microtitre plate. Some cells were left in media only to act as cellular controls. The methanol and poly-L-lysine were negative controls and the dextran was a positive control. The cells were left in the incubator for 72 hours, and checked occasionally for yeast or bacterial contamination. Five hours prior to the incubation time end point, at 67 hours, 20 μl ofMTT was added and the cells left for the final 5 hours. Then cellular media was removed, 100 μl of optical grade DMSO (Sigma) was added and the MTT crystals dissolved. The plates were read in a Titerteck plate reader and the results (OD) are expressed in FIGs. 3 and 4 as a percentage of the OD seen in cell wells containing no polymer or control.

Result

Cationic dendrimers were cytotoxic (similar to poly-L-lysine) towards the cell line, while anionic dendrimers (including PAMAM generation 3.5, EDA core) were not cytotoxic (similar to dextran).

Example 3 Cell Cytotoxicity of unmodified dendrimers against CCRF-CEM cells

Method

CCRF-CEM cells are lymphoblastic leukemia and a suspension cell line, *i.e.* it grows in suspension. CCRF-CEM cells were seeded at a density of 5 x $10^4$ cells per ml (5 x $10^3$ cells per well) in a 96 well V-shape microtitre plate (Costar) in RPMI 1640 tissue culture media (Gibco) supplemented with 10% FCS (Gibco). All cellular growth and cytotoxic incubations were carried out in a cell incubator at 37°C and 5% $CO_2$ atmosphere.

Cell density was assessed using an improved neurenbrow hemocytometer (Sigma). The cells were centrifuged at 1000 x g and resuspended in fresh media (supplemented with FCS) before the cell density was assessed. The cells were then seeded in a microtitre plate. The cells were left for 24 hours to recover and re-adhere.

All polymers and controls were dissolved in RPMI media (supplemented with FCS) and then sterilized through a 0.2 μm sterile filter (Acrodisk), the first few microliters of the solution being discarded in the case of adherence of the polymer to the filter membrane. Polymer and controls were then added in increasing concentrations to the cells in the microtitre plate. Some cells were left in media only to act as cellular controls. The methanol and poly-L-lysine were negative controls and the dextran was a positive control. The cells were left in the incubator for 72 hours, and checked occasionally for yeast or bacterial contamination. Five hours prior to the incubation time end point, at 67 hours, 20 μl MTT was added, and the cells left for the final 5 hours. Then cellular media was removed, 100 μl of optical grade DMSO (Sigma) was added and the MTT crystals dissolved. The plates were read in a Titerteck plate reader and the results (OD) are expressed in FIGs. 5 and 6 as a percentage of the OD seen in cell wells containing no polymer or control

Result

Cationic dendrimers were cytotoxic (similar to poly-L-lysine) towards the cell line, while anionic dendrimers (including PAMAM generation 3.5) were not cytotoxic (similar to dextran).

Example 4 Cell Cytotoxicity of unmodified dendrimers against HepG2 cells

Method

HepG2 is a hepatocellular carcinoma and is an adherent cell line, *i.e.* it grows in a monolayer. HepG2 cells were seeded at a density of 1 x $10^5$ cells per ml (1 x $10^4$ cells per well) in a 96 well flat bottomed microtitre plate (Costar)

in MEM tissue culture media (Gibco) supplemented with 10% FCS (Gibco). All cellular growth and cytotoxic incubations were carried out in a cell incubator at 37°C and 5% $CO_2$ atmosphere.

Cell density was assessed using an improved neurenbrow hemocytometer (Sigma). The cells were washed with PBS twice and fresh RPMI media (supplemented with FCS) added, the cells were then seeded in a microtitre plate. The cells were left for 24 hours to recover and re-adhere.

All polymers and controls were dissolved in RPMI media (supplemented with FCS) and then sterilized through a 0.2 μm sterile filter (Acrodisk), the first few microliters of the solution being discarded in the case of adherence of the polymer to the filter membrane. Polymer and controls were then added in increasing concentrations to the cells in the microtitre plate. Some cells were left in media only to act as cellular controls. The methanol and poly-L-lysine were negative controls and the dextran was a positive control. The cells were left in the incubator for 72 hours, and checked occasionally for yeast or bacterial contamination. Five hours prior to the incubation time end point, at 67 hours, 20 μl of MTT was added and the cells left for the final 5 hours. The cellular media was removed and 100 μl of optical grade DMSO (Sigma) was added and the MTT crystals dissolved. The plates were read in a Titerteck plate reader and the results (OD) are expressed in FIGs. 7 and 8 as a percentage of the OD seen in cell wells containing no polymer or control.

Result

Cationic dendrimers were cytotoxic (similar to poly-L-lysine, MW 56 KDa) towards the cell line, while anionic dendrimers (including PAMAM gen. 3.5) were not cytotoxic (similar to dextran, MW 78 KDa).

Example 5A Synthesis of an encapsulated cisplatin dendritic polymer conjugate

Method

0.8207 Gram (6.40 x $10^{-5}$ mol) of poly(amidoamine)Starburst® dendrimer (generation 3.5, EDA core with carboxylate surface groups) was dissolved in 20 ml of 18Ω water (Barnstead-Nanopure). 0.6601 Gram (2.22 x $10^{-3}$ mol) of cisplatin was dissolved in 300 ml of 18Ω water (Barnstead-Nanopure). Once the cisplatin was fully dissolved in the water, the dendrimer was added dropwise to the stirred solution of cisplatin, under nitrogen, over a period of 45 minutes (a molar ratio of cisplatin to dendrimer of approximately 35:1.). The solution was isolated from light and stirred under nitrogen for 24 hours at room temperature. The unreacted or excess cisplatin was removed using Centricon Plus-80 MWCO 5000 (Millipore, Bioseparations). Upon recovery of the retentate, the isolated solution was lyophilized for 48 hours (The Labconco FreeZone 4.5 Lit.) to give the hygroscopic dendrimer-platinate as a fine white powder.

Results

The weight percent was determined at 19.25 wt% Pt.

Example 5B Synthesis of an encapsulated carboplatin dendritic polymer conjugate

Method

0.6964 Grams (5.39 x $10^{-5}$ mol) of poly(amidoamine) Starburst® dendrimer (generation 3.5, EDA core) was dissolved in 20 ml of 18Ω water (Barnstead-Nanopure). 0.666 g (1.79 x $10^{-3}$ mol) of carboplatin was dissolved in 300 ml 18Ω water (Barnstead-Nanopure). This mixture was gently heated (35°C) until the carboplatin was completely in solution. Once the carboplatin was fully dissolved in the water, the dendrimer was added dropwise, under nitrogen, and stirred over a period of 45 minutes (a molar ratio of carboplatin to dendrimer of 33:1). The solution was isolated from light and stirred under nitrogen at room temperature for 24 hours. The unreacted or excess carboplatin was removed using Centricon Plus-80 MWCO 5000 (Millipore, Bioseparations). Upon recovery of the retentate, the isolated solution was lyophilized for 48 hours (The Labconco FreeZone 4.5 Lit.) to give the hygroscopic dendrimer-platinate as a fine white powder.

Results

The weight percent was determined at 20.47 wt% Pt.

Example 6 Generational Affect

Method

Poly(amidoamine) Starburst® dendrimers, EDA core, of generation 3.5, 4.5, and 5.5 with sodium carboxylic surface groups were each dissolved in 20 ml of 18Ω water (Barnstead-Nanopure) and then were reacted with cisplatin. A molar ratio of cisplatin to dendrimer of 33:1, 74:1, 127:1, respectively was used. The cisplatin was dissolved in 300ml of 18Ω water (Barnstead-Nanopure) and gently heated (35°C) until the cisplatin was completely in solution. Once the cisplatin was fully dissolved in the water, the dendrimer was added dropwise, under nitrogen, and stirred over a period of 45 minutes. The solution was isolated from light and stirred under nitrogen at room temperature for a reaction time of 24 hours. The unreacted or excess cisplatin was removed using Centricon Plus-80 MWCO 5000 (Millipore, Bioseparations). Upon recovery of the of retentate, the isolated solution was lyophilized for 48 hours (The Labconco FreeZone 4.5 Lit.) to give the hygroscopic dendrimer-platinate encapsulate as a fine white powder.

Results

The weight percent was determined at 19.25, 16.82, 16.81 wt% Pt for the 3.5, 4.5, and 5.5 poly(amidoamine) generation dendrimers, respectively. The data appears to suggest that the increased generations slightly lowers the rate of platin loading in the dendrimer-platin conjugates probably because of the compact (physical crowding) surface groups. However, even with the use of a poly(amidoamine) dendrimer of generation 5.5 with sodium carboxylate surface groups a loading of 16.81 wt% Pt is obtained. Considering the molecular weight of the PAMAM dendrimers (generation 3.5, MW 12931; 4.5, MW 26252; and 5.5, MW 52913), the weight percent in terms of molar ratio indicates an increased uptake of platin from lower to higher generations.

Example 7 Kinetic study of reaction procedure

Method

Reactions were carried out where a poly(amidoamine) Starburst® dendrimer of generation 3.5 with sodium carboxylate surface groups were dissolved in 20 ml of 18Ω water (Barnstead-Nanopure) and reacted with cisplatin at a molar ration of 32:1. The cisplatin was dissolved in 300ml of 18Ω water (Barnstead-Nanopure) and gently heated (35°C) until the cisplatin was completely in solution. Once the cisplatin was fully dissolved in the water, the dendrimer was added dropwise, under nitrogen, and stirred over a period of 45 minutes. The solution was isolated from light and stirred under nitrogen at room temperature for reaction times of 4hours, 24hours, and 48 hours. The unreacted or excess cisplatin was removed using Centricon Plus-80 MWCO 5000 (Millipore, Bioseparations). Upon recovery of the retentate, the isolated solution was lyophilized for 48 hours (The Labconco FreeZone 4.5 Lit.) to give the hygroscopic dendrimer-platinate encapsulate as a fine white powder.

Results

The weight percent was determined at 5.81, 19.25, 20.26 wt% Pt for the reaction times of 4, 24 and 48 hours, respectively. The data appears to suggest that the longer reaction times (*i.e.,* 24 hours versus 4 hours) favors a higher platin loading in the dendrimer-platin conjugates. However, when the reaction time was increased to 48 hours, platin loading remained essentially unchanged. This indicates that the optimum loading is reached using a 24 hour reaction time.

Example 8 Modification of Surface Functionalities

Method

Poly(amidoamine) Starburst® dendrimers of generation 3.5 with varying surface groups were dissolved in 20 ml of 18Ω water (Barnstead-Nanopure) and were reacted with cisplatin. A molar ratio of cisplatin to dendrimer of 32:1, 16:1, 32:1, respectively was used and surface groups consisted of amine groups, acetamide groups, and extended carboxylic groups, respectively. The extended carboxylic groups were prepared by reacting the generation 3 amine groups with succinic anhydride in DMSO. This involved the formation of an amide goup on the surface amine (*e.g.*, from methylmethacrylate) with a concurrent ring opening of the succinic anhydride to produce extended carboxylic acid groups (*e.g.*, carboxylated surface). The cisplatin was dissolved in 300ml of 18Ω water (Barnstead-Nanopure) and gently heated (35°C) until the cisplatin was completely in solution. Once the cisplatin was fully dissolved in the water, the dendrimer was added dropwise, under nitrogen, and stirred over a period of 45 minutes. The solution was isolated from light and

stirred under nitrogen at room temperature for a reaction time of 24 hours. The unreacted or excess cisplatin was removed using Centricon Plus-80 MWCO 5000 (Millipore, Bioseparations). Upon recovery of the of retentate, the isolated solution was lyophilized for 48 hours (The Labconco FreeZone 4.5 Lit.) to give the hygroscopic dendrimer-platinate encapsulate as a fine white powder.

Results

These conjugates resulted in very low platin loading, 6.25, 0.98, and, 0.01 wt% Pt, respectively. These data suggest that the first interior amine groups, as well as the carboxylate groups on the surface of the dendrimer, may play a significant role in the uptake of the platinate.

Example 9 Purification and Reproducibility

Method

The reaction of cisplatin with dendrimers appears to still contain unbound cisplatin, even after several hours reaction time. Therefore, as a part of the synthetic procedure in the preparation of dendrimer-platinates, the unbound cisplatin must be removed. Operating on the general premise that the cisplatin was covalently attached to the dendrimer (however later known not to be the case in several preparations) a number of commercially available ultrafiltration devices were tested as a means to remove the unbound cisplatin. Specifically tested were: (1) an Amicon stirred cell with a 3000 MWCO or a 10000 MWCO membrane where fluid is forced through the membrane under nitrogen pressure, (2) Amicon Centriprep devices with 3000 MWCO filters where fluid is forced through the membrane by centrifugation, and (3) Amicon Centricon plus devices with 5000 MWCO filters where fluid is forced through the membrane by centrifugation. Theoretically, the retentate can be repeatedly washed in these devices thus continuously diluting out any remaining cisplatin. The dendrimer-cisplatin conjugates of this example were synthesized in a similar manner as before, and all the reaction reported used the same lot of dendrimer, the same ration of cisplatin to dendrimer, and the same reaction times.

Results

The retention of cisplatin by the dendrimer decreased with increased purification times and increased wash volumes. The results are summarized in the table below. The results indicate that the weight percent of platinum found in the final product depends on the filtration technique, as well as the volume of solvent used to wash the retentate in the purification technique. The practical significance of these date is as follows: (1) the data strongly suggest that there is a significant loss of platin from the conjugate during the purification process, thereby indicating that the platin is not irreversibly or covalently bound to the dendrimer; and (2) the loss of platin during filtration may roughly approximate the rate of release. This result is consistent with a reversible encapsulation of the cisplatin rather than a surface attachment.

Influence on the purification technique on Pt loading of the dendrimer-platinate

| Weight of Dendrimer | Purification | Yield (wt%) | Wt% Pt |
|---|---|---|---|
| 0.0757 | 2.66 hrs, CTP3K, no wash | 74.8 | 6.96 |
| 0.1245 | 5-6 hrs, CTP3K, 93-103 ml wash | 60.7 | 3.18 |
| 0.2565 | 8 hrs, SC3K, 580 ml wash | 59.4 | 2.34 |
| 0.2285 | 22.77 hrs, SC10K, 1525 ml wash | 65.2 | 0.64 |
| SC = Stirred Cell, CTP = Centiprep | | | |

It also appears from these data that a fast wash of the conjugate does remove the unbound surface Pt and perhaps also some bound surface Pt; however, a long wash will cause the encapsulated Pt to be removed from the interior of the dendrimer.

Example 13A *In vitro* release of platinum from the dendrimer-platinate in biological fluids

Method

Known amounts of cisplatin and dendrimer were placed in two buffered solutions 25 (PBS at pH 7.4 and Citrate-Phosphate at pH 5.5) to simulate different biological compartments (the plasma/extracellular and the lysosomal compartments, respectively). The solution was sealed in a dialysis bag with a MWCO of 10 KD. Then the bag was placed in a container filled with the respective buffered solution. The solutions were then placed in heated water bath at 37°C. At regular intervals, samples from the buffer solutions were removed and analyzed in triplicate (over a period of 74 hours). At the end of the experiment, a sample was taken from within the bag. All the samples were analyzed using atomic absorption spectroscopy as described previously.

Result

The amount of platinum released at pH 5.5 was slightly greater than that released at pH 7.4. However, as shown in FIG. 11, the total amount released over time remained less than 1 % of the total.

Example 13B Cell Cytotoxicity of dendrimer-platinate (B16F10, L1210, CorL23)

Method

Cells were seeded at a density of 1 x $10^5$ cells per ml (1 x $10^4$ cells per well) in a 96 well flat bottomed microtitre plate (Costar) in RPMI 1640 tissue culture media (Gibco) supplemented with 10% FCS (Gibco). All cellular growth and cytotoxic incubations were carried out in a cell incubator at 37°C and 5% $CO_2$ atmosphere.

Cell density was assessed using an improved neurenbrow hemocytometer (Sigma). The cells were washed with PBS twice and fresh RPMI media (supplemented with FCS) added, the cells were then seeded in a microtitre plate. The cells were left for 24 hours to recover and re-adhere. If cells were in a suspension they were spun at 1000 x g and resuspended in fresh media.

The dendrimer-platinate and cisplatin were dissolved in RPMI media (supplemented with FCS) and then sterilized through a 0.2 μm sterile filter (Acrodisk), the first few microlitres of the solution being discarded in the case of adherence of the polymer to the filter membrane. Then dendrimer and cisplatin were added in increasing concentrations to the cells in the microtitre plate. Some cells were left in media only to act as cellular controls. The cells were left in the incubator for 72 hours, and checked occasionally for yeast or bacterial contamination. Five hours prior to the incubation time end point, at 67 hours, 20 μl of MTT was added and the cells left for the final 5 hours. Then cellular media was removed and 100 μl of optical grade DMSO (Sigma) was added and the MTT crystals dissolved. The plates were read in a Titerteck plate reader and the results (OD) are expressed in FIGs. 12, 13 and 14 as a percentage of the OD seen in cell wells containing no dendrimer-platinate or cisplatin.

Result

The dendrimer-platinate was less cytotoxic than the cisplatin alone by several orders of magnitude.

Example 14 Pharmacology (I.P. Tumor verses I.P. Injection)

Method

Li 210 or B16F10 cells were injected at a cell density into a mouse (DBA2 or C57 respectively, 25g) at a cell density of 1 x $10^5$ (0.9% saline solution) into the intraperitoneal (IP. 100 μl) cavity. Twenty-four hours later, the dendrimer-platinate and cisplatin (on one day or on three consecutive days) were injected at a concentration according to the weight of the mouse (*e.g.* 1 mg/kg - 15 mg/kg). The mouse body weight and general toxicity was also monitored according to UK guidelines in the use of animals used in neoplasia studies. At the end point the gross morphology of the organs was noted.

Result

This pharmacology demonstrated the maximum tolerated does of the dendrimer-platinate (25-50 mg/kg). As shown in FIG. 10, I.P. delivery of dendrimer platinate showed anti-tumor activity but not substantially better than cisplatin alone.

Example 15 Pharmacology (S.C. Tumor verses I.V. Injection)

Method

B16F10 cells were injected at a cell density of 1 x $10^5$ (0.9% saline solution) into the left or right flank of the C57 mouse S.C. The mouse was then left until the tumor was visible at a palpable size of between 50 - 100 $mm^2$. Then the platin-based analogue dendrimer and cisplatin were injected I.V. into the tail vein at the respective doses. The animal was monitored and the tumor size measured using calipers and recorded on a daily basis. When the animal tumor size was between 300 - 400 $mm^2$, the animal was culled. The tumor excised and weighed and gross morphology of the organs noted.

Result

The platin-based analogue dendrimer was active against the S.C. tumor and demonstrated a significant difference in the final tumor weight and survival time, as shown in FIG. 11.

Example 16 Biodistribution of platin-based analogue dendrimer *in vivo*

Method

C57 mice were injected S.C. with B16F10 cells at a cell density of 1 x $10^5$ cells per mouse. The tumor was allowed to reach a palpable size before injecting the platin-based analogue dendrimer I.V. At specific time points (0 - 24 hours) the animal was culled and key organs (liver, kidney, and blood) including the tumor were isolated and weighed. The organs were solubilized in concentrated nitric acid (10M) and hydrogen peroxide added to decolorize the solution during boiling. The solutions were made up to a fixed volume (25 ml) and then analyzed using AAS after addition of lanthanum (La) (excess) to free up bound platinum (Pt).

Result

Compared to cisplatin alone, the platin-based analogue dendrimer was found to accumulate preferentially in the tumor by at least 3x, relatively quickly after the injection. The results are shown in FIG. 12.

Conclusion

Antineoplastic dendritic polymer conjugates have greater water solubility than cisplatin, were 3-5 times less toxic, and have greater anti-tumor activity *in vivo*.

Although the invention has been described with reference to its preferred embodiments, those of ordinary skill in the art may, upon reading and understanding this disclosure, appreciate changes and modifications which may be made which do not depart from the scope of the invention as described above or claimed hereafter.

## Claims

1. An antineoplastic dendritic polymer conjugate, comprising an antineoplastic agent which is a platinum, titanium, vanadium, niobium, molybdenum, rhenium, or tin metal containing analogue compound of an antineoplastic agent, encapsulated within a dendritic polymer containing anionic functional terminal groups.

2. The antineoplastic dendritic polymer conjugate of claim 1, wherein said antineoplastic agent is a compound having a tetravalent platinum atom bonded to the nitrogen atom of two amine ligands, which may be the same or different, the amine ligands being in cis conformation in respect to each other.

3. The antineoplastic dendritic polymer conjugate of claim 1, wherein said antineoplastic agent is cisplatin, carboplatin, oxalipatin, teraplatin,platinum-DACH,ormaplatin, titanocene dichloride, vanadocene dichloride, niobocene dichloride, molybdenocene dichloride, rhenocene dichloride, a diorganotindihalide or another metal containing antineoplastic agent.

4. The antineoplastic dendritic polymer conjugate of claim 1 or 3, wherein the antineoplastic agent is a platin-based analogue.

5. The antineoplastic dendritic polymer conjugate of claim 4, wherein said platin-based analogue is cisplatin or carboplatin.

6. The antineoplastic dendritic polymer conjugate of claim 1, wherein the antineoplastic agent can be conjugated to the surface of the dendritic polymer with a linker.

7. The antineoplastic dendritic polymer conjugate of claim 4, wherein the molar ratio of said platin-based analogue to dendritic polymer is from about 100: 1 to about 1: 1.

8. The antineoplastic dendritic polymer conjugate of claim 7, wherein the molar ratio of said platin-based analogue to dendrimer is about 35 : 1.

9. The antineoplastic dendritic polymer of claim 1, wherein said dendritic polymer contains caboxylic acid terminal groups.

10. The antineoplastic dendritic polymer conjugate of claim 1, 3, 5, 6, or 9, wherein said dendritic polymer is a dendrimer.

11. The antineoplastic dendritic polymer conjugate of claim 1 or 9, wherein said dendrimer is a poly (amidoamine) or poly (propyleneimine) dendrimer.

12. The antineoplastic dendritic polymer conjugate of claim 11, wherein the dendrimer is a poly (amidoamine) dendrimer of a generation from 3.5 to 7.5.

13. A method for preparing an antineoplastic dendritic polymer conjugate, comprising:

providing a dendritic polymer having functional groups which are accessible to an antineoplastic agent capable of interacting with said functional groups in a suitable solvent;
contacting the dendritic polymer with an antineoplastic agent in a suitable solvent under conditions sufficient to cause the antineoplastic agent to associate with the dendritic polymer; and
allowing the antineoplastic agent sufficient time to be encapsulated within the dendritic polymer.

14. The method of claim 13, wherein the antineoplastic agent is a platin-based analogue.

15. The method of claim 14, wherein the platin-based analogue is cisplatin or carboplatin.

16. The method of any one of claims 13-15, wherein the dendritic polymer is a dendrimer.

17. The method of claim 16, wherein the dendritic polymer is a poly (amidoamine) or a poly (propyleneimine) dendrimer.

18. The method of claim 13, wherein the functional terminal groups are carboxylic acids.

19. The method of claim 13, wherein the dendritic polymer is dissolved in water and is contacted with an antineoplastic agent dissolved in water.

20. The method of claim 13, wherein the antineoplastic agent is a platin-based analogue with a molar ratio to the dendritic polymer in the conjugate from about 100: 1 to 1:1.

21. The method of claims 20, wherein the molar ratio of the platin-based analogue to the dendritic polymer in the conjugate is from about 35: 1.

22. The method of claim 20 or 21, wherein the platin-based analogue is cisplatin or carboplatin.

**Patentansprüche**

1. Antineoplastisches dendritisches Polymerkonjugat, umfassend ein antineoplastisches Mittel, welches ein Platin-, Titan-, Vanadium-, Niobium-, Molybdän-, Rhenium-, oder Zinnmetall enthaltendes Analogon eines antineoplastischen Mittels ist und welches in einem anionische funktionelle Endgruppen enthaltenden dendritischen Polymer

verkapselt ist.

2. Antineoplastisches dendritisches Polymerkonjugat nach Anspruch 1, wobei das antineoplastische Mittel eine Verbindung ist, in welcher ein vierbindiges Platinatom an das Stickstoffatom zweier Aminliganden, welche gleich oder verschieden sein können, gebunden ist, wobei die Aminliganden in *cis*-Konformation zueinander vorliegen.

3. Antineoplastisches dendritisches Polymerkonjugat nach Anspruch 1, wobei das antineoplastische Mittel Cisplatin, Carboplatin, Oxaliplatin, Teraplatin, Platin-DACH, Ormaplatin, Titanocendichlorid, Vanadocendichlorid, Niobocendichlorid, Molybdänocendichlorid, Rhenocendichlorid, ein Diorganozinndihalogenid, oder ein anderes metallhaltiges antineoplastisches Mittel ist.

4. Antineoplastisches dendritisches Polymerkonjugat nach Anspruch 1 oder 3, wobei das antineoplastische Mittel ein auf Platin basierendes Analogon ist.

5. Antineoplastisches dendritisches Polymerkonjugat nach Anspruch 4, wobei das auf Platin basierende Analogon Cisplatin oder Carboplatin ist.

6. Antineoplastisches dendritisches Polymerkonjugat nach Anspruch 1, wobei das antineoplastische Mittel über einen Linker an die Oberfläche des dendritischen Polymers konjugiert sein kann.

7. Antineoplastisches dendritisches Polymerkonjugat nach Anspruch 4, wobei das Molverhältnis von auf Platin basierendem Analogon zu dendritischem Polymer etwa 100:1 bis etwa 1:1 beträgt.

8. Antineoplastisches dendritisches Polymerkonjugat nach Anspruch 7, wobei das Molverhältnis von auf Platin basierendem Analogon zu Dendrimer etwa 35:1 beträgt.

9. Antineoplastisches dendritisches Polymer nach Anspruch 1, wobei das dendritische Polymer Carbonsäureendgruppen enthält.

10. Antineoplastisches dendritisches Polymerkonjugat nach Anspruch 1, 3, 5, 6 oder 9, wobei das dendritische Polymer ein Dendrimer ist.

11. Antineoplastisches dendritisches Polymerkonjugat nach Anspruch 1 oder 9, wobei das Dendrimer ein Poly(amidoamin)- oder Poly(propylenimin)-Dendrimer ist.

12. Antineoplastisches dendritisches Polymerkonjugat nach Anspruch 11, wobei das Dendrimer ein Poly(amidoamin)-Dendrimer einer Generation von 3.5 bis 7.5 ist.

13. Verfahren zur Herstellung eines antineoplastischen dendritischen Polymerkonjugats, umfassend:

    Bereitstellen eines dendritischen Polymers mit funktionellen Gruppen, welche für ein antineoplastisches Mittel zugänglich sind, das mit den funktionellen Gruppen in einem geeigneten Lösungsmittel wechselwirken kann,
    Inkontaktbringen des dendritischen Polymers mit einem antineoplastischen Mittel in einem geeigneten Lösungsmittel unter Bedingungen, welche ausreichend sind, um eine Assoziation des antineoplastischen Mittels mit dem dendritischen Polymer zu bewirken, und
    Zugestehen einer ausreichenden Zeitdauer, um eine Verkapselung des antineoplastischen Mittels in dem dendritischen Polymer zu gestatten.

14. Verfahren nach Anspruch 13, wobei das antineoplastische Mittel ein auf Platin basierendes Analogon ist.

15. Verfahren nach Anspruch 14, wobei das auf Platin basierende Analogon Cisplatin oder Carboplatin ist.

16. Verfahren nach einem der Ansprüche 13-15, wobei das dendritische Polymer ein Dendrimer ist.

17. Verfahren nach Anspruch 16, wobei das dendritische Polymer ein Poly(amidoamin)- oder Poly(propylenimin)-Dendrimer ist.

18. Verfahren nach Anspruch 13, wobei die funktionellen Endgruppen Carbonsäuren sind.

**19.** Verfahren nach Anspruch 13, wobei das dendritische Polymer in Wasser gelöst wird und mit einem in Wasser gelösten antineoplastischen Mittel in Kontakt gebracht wird.

**20.** Verfahren nach Anspruch 13, wobei das antineoplastische Mittel ein auf Platin basierendes Analogon ist, welches im Konjugat in einem Molverhältnis von etwa 100:1 1 bis 1:1 1 zum dendritischen Polymer vorliegt.

**21.** Verfahren nach Anspruch 20, wobei das Molverhältnis von auf Platin basierendem Analogon zum dendritischen Polymer im Konjugat etwa 35:1 beträgt.

**22.** Verfahren nach Anspruch 20 oder 21, wobei das auf Platin basierende Analogon Cisplatin oder Carboplatin ist.

**Revendications**

**1.** Conjugué de polymère dendritique et d'agent antinéoplasique, comprenant un agent antinéoplasique, qui est un composé analogue d'un agent antinéoplasique et contenant un métal, platine, titane, vanadium, niobium, molybdène, rhénium ou étain, encapsulé dans un polymère dendritique contenant des groupes fonctionnels terminaux anioniques.

**2.** Conjugué de polymère dendritique et d'agent antinéoplasique, conforme à la revendication 1, dans lequel ledit agent antinéoplasique est un composé comportant un atome de platine tétravalent lié aux atomes d'azote de deux ligands amine qui peuvent être identiques ou différents, ces ligands amine étant en conformation cis l'un par rapport à l'autre.

**3.** Conjugué de polymère dendritique et d'agent antinéoplasique, conforme à la revendication 1, dans lequel ledit agent antinéoplasique est du cisplatine, du carboplatine, de l'oxaliplatine, du téraplatine, du DACH-platine, de l'ormaplatine, du dichlorure de titanocène, du dichlorure de vanadocène, du dichlorure de niobocène, du dichlorure de molybdénocène, du dichlorure de rhénocène, un dihalogénure de diorgano-étain, ou un autre agent antinéoplasique contenant un métal.

**4.** Conjugué de polymère dendritique et d'agent antinéoplasique, conforme à la revendication 1 ou 3, dans lequel l'agent antinéoplasique est un analogue à base de platine.

**5.** Conjugué de polymère dendritique et d'agent antinéoplasique, conforme à la revendication 4, dans lequel ledit analogue à base de platine est du cisplatine ou du carboplatine.

**6.** Conjugué de polymère dendritique et d'agent antinéoplasique, conforme à la revendication 1, dans lequel l'agent antinéoplasique peut être conjugué à la surface du polymère dendritique au moyen d'un raccord.

**7.** Conjugué de polymère dendritique et d'agent antinéoplasique, conforme à la revendication 4, dans lequel le rapport molaire dudit analogue à base de platine au polymère dendritique vaut d'environ 100/1 à environ 1/1.

**8.** Conjugué de polymère dendritique et d'agent antinéoplasique, conforme à la revendication 7, dans lequel le rapport molaire dudit analogue à base de platine au dendrimère vaut à peu près 35/1.

**9.** Conjugué de polymère dendritique et d'agent antinéoplasique, conforme à la revendication 1, dans lequel ledit polymère dendritique contient des groupes terminaux de type acide carboxylique.

**10.** Conjugué de polymère dendritique et d'agent antinéoplasique, conforme à la revendication 1, 3, 5, 6 ou 9, dans lequel ledit polymère dendritique est un dendrimère.

**11.** Conjugué de polymère dendritique et d'agent antinéoplasique, conforme à la revendication 1 ou 9, dans lequel ledit dendrimère est un dendrimère de type poly(amidoamine) ou poly(propylène-imine).

**12.** Conjugué de polymère dendritique et d'agent antinéoplasique, conforme à la revendication 11, dans lequel le dendrimère est un dendrimère de type poly(amidoamine) de génération 3,5 à 7,5.

**13.** Procédé de préparation d'un conjugué de polymère dendritique et d'agent antinéoplasique, comprenant les étapes suivantes :

- prendre un polymère dendritique comportant des groupes fonctionnels qui soient accessibles à un agent antinéoplasique capable d'interagir avec ces groupes fonctionnels dans un solvant approprié ;
- mettre le polymère dendritique au contact d'un agent antinéoplasique dans un solvant approprié, dans des conditions appropriées pour amener l'agent antinéoplasique à s'associer au polymère dendritique ;
- et laisser suffisamment de temps à l'agent antinéoplasique pour qu'il s'encapsule dans le polymère dendritique.

**14.** Procédé conforme à la revendication 13, dans lequel l'agent antinéoplasique est un analogue à base de platine.

**15.** Procédé conforme à la revendication 14, dans lequel l'analogue à base de platine est du cisplatine ou du carboplatine.

**16.** Procédé conforme à l'une des revendications 13 à 15, dans lequel le polymère dendritique est un dendrimère.

**17.** Procédé conforme à la revendication 16, dans lequel le polymère dendritique est un dendrimère de type poly (amidoamine) ou poly(propylène-imine).

**18.** Procédé conforme à la revendication 13, dans lequel les groupes fonctionnels terminaux sont des groupes de type acide carboxylique.

**19.** Procédé conforme à la revendication 13, dans lequel le polymère dendritique est dissous dans de l'eau et mis au contact d'un agent antinéoplasique dissous dans de l'eau.

**20.** Procédé conforme à la revendication 13, dans lequel l'agent antinéoplasique est un analogue à base de platine qui se trouve, dans le conjugué, en un rapport molaire au polymère dendritique d'environ 100/1 à environ 1/1.

**21.** Procédé conforme à la revendication 20, dans lequel le rapport molaire de l'analogue à base de platine au polymère dendritique, dans le conjugué, vaut à peu près 35/1.

**22.** Procédé conforme à la revendication 20 ou 21, dans lequel l'analogue à base de platine est du cisplatine ou du carboplatine.

# FIG. 1

Effect of Cationic Dendrimers on Haemolysis
of rat erythrocytes, 1h

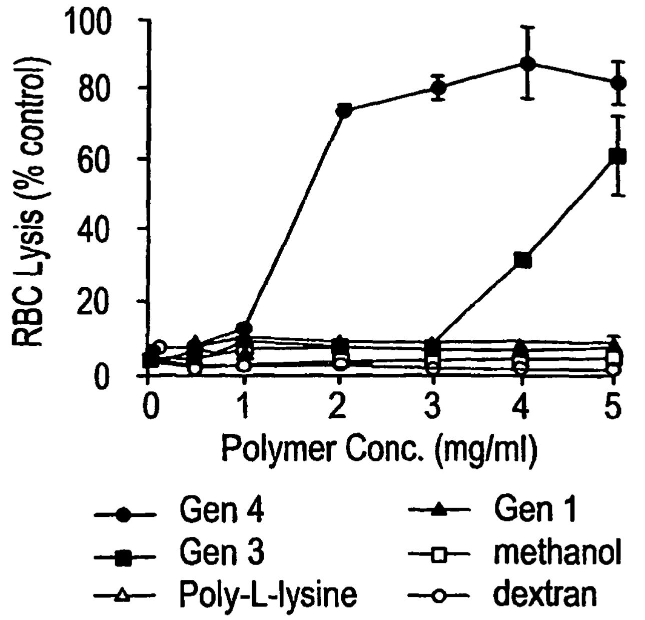


# FIG. 2

Effect of Anionic Dendrimers on Haemolysis of rat erythrocytes, 1h

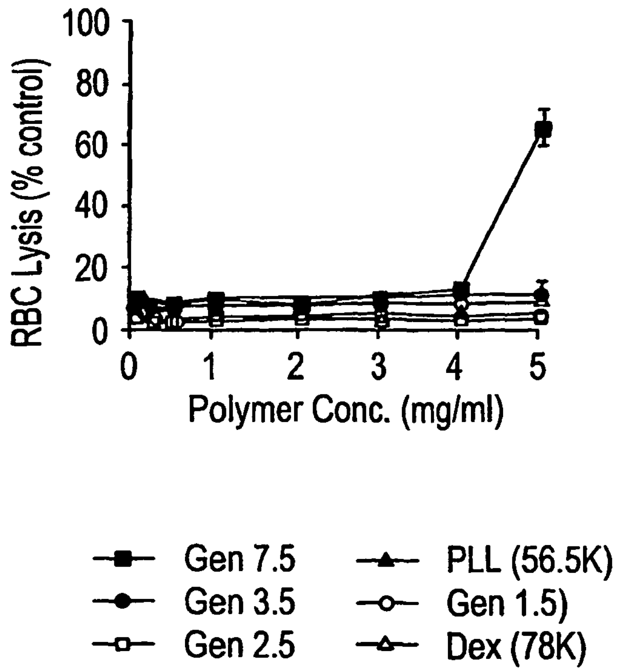

# FIG. 3

Effect of Anionic Dendrimers on B16F10, 72h

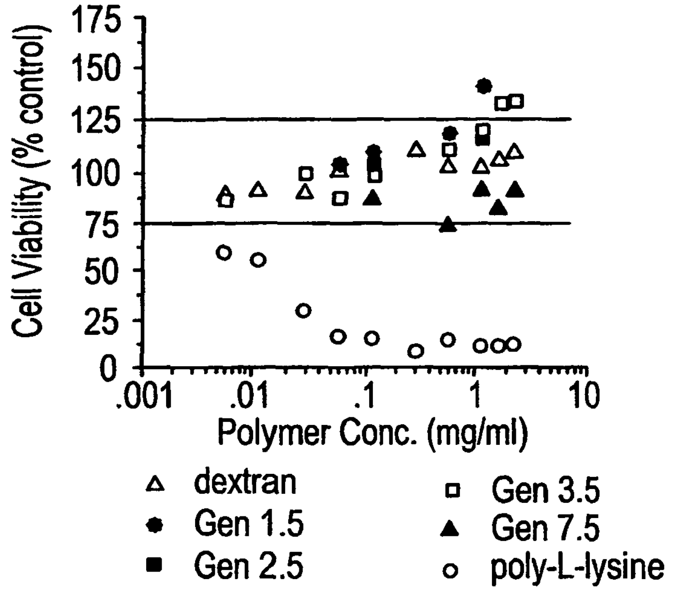


# FIG. 4

Effect of Cationic Dendrimers on B16F10, 72h

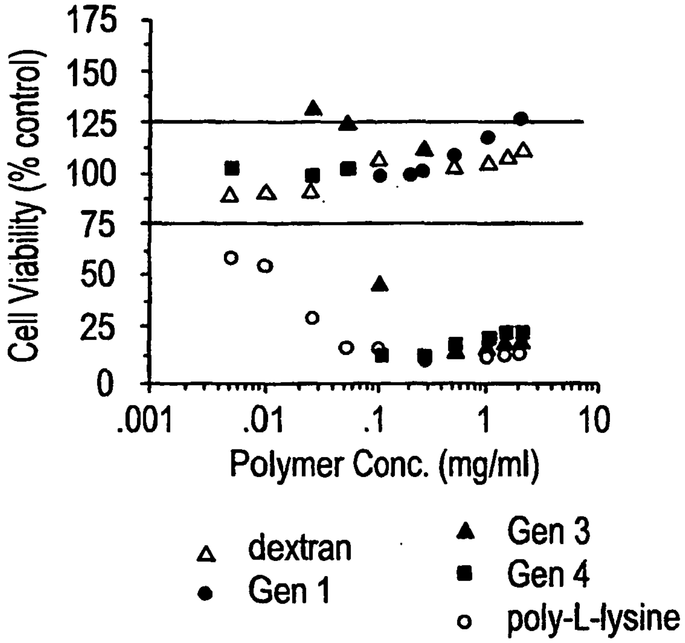

# FIG. 5

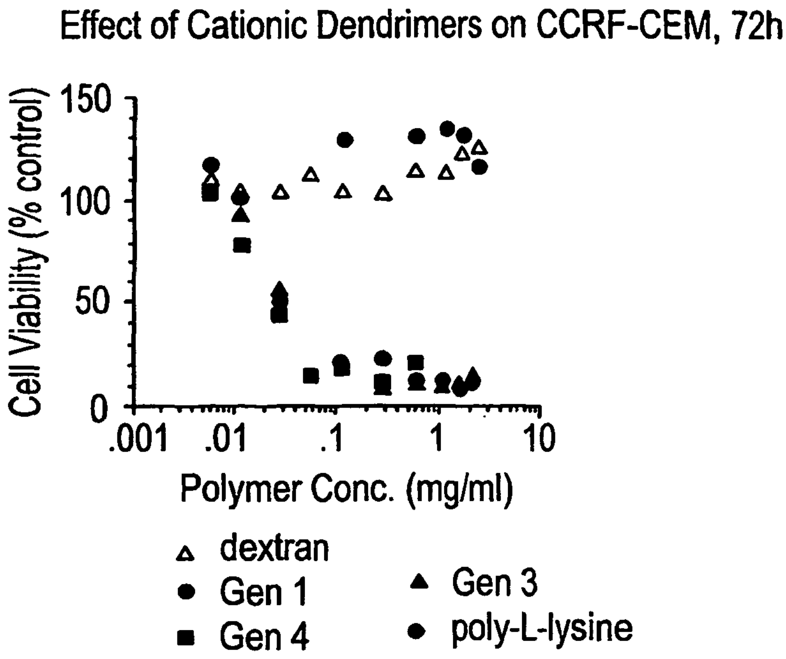
Effect of Cationic Dendrimers on CCRF-CEM, 72h
Cell Viability (% control)
150
100
50
0
.001
.01
.1
1
10
Polymer Conc. (mg/ml)
dextran
Gen 1
Gen 4
Gen 3
poly-L-lysine

# FIG. 6

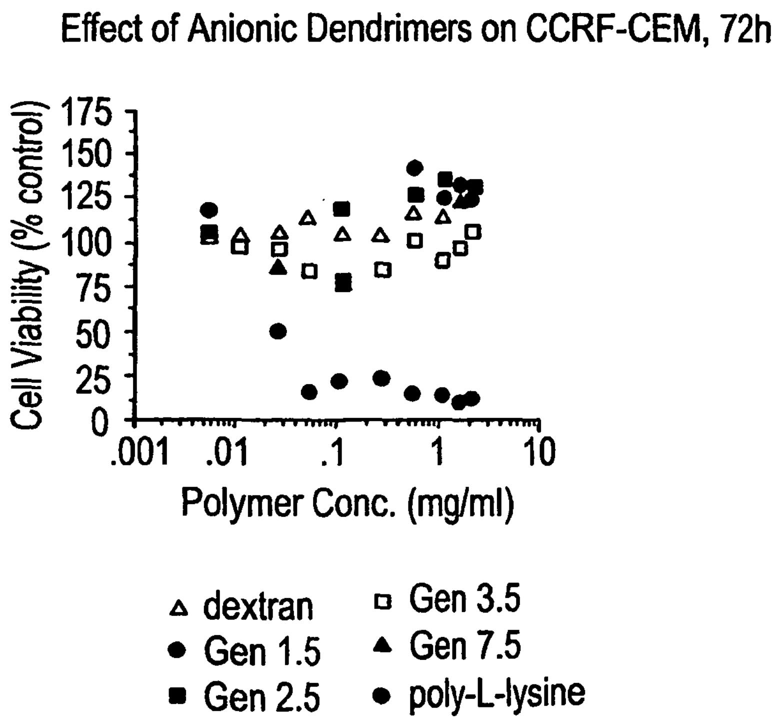
Effect of Anionic Dendrimers on CCRF-CEM, 72h
Cell Viability (% control)
175
150
125
100
75
50
25
0
.001
.01
.1
1
10
Polymer Conc. (mg/ml)
dextran
Gen 1.5
Gen 2.5
Gen 3.5
Gen 7.5
poly-L-lysine

FIG. 7

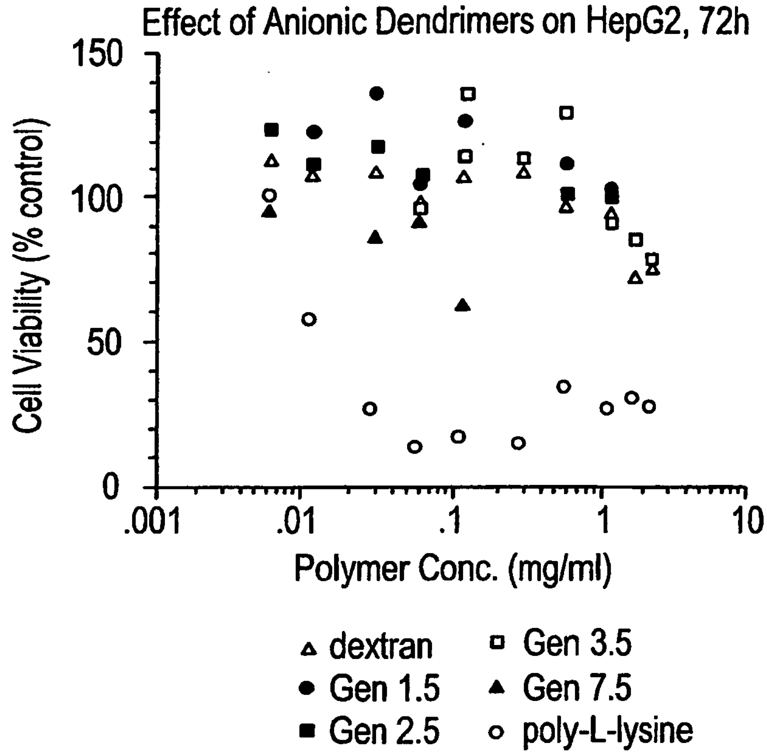
Effect of Anionic Dendrimers on HepG2, 72h
150
100
50
0
Cell Viability (% control)
.001
.01
.1
1
10
Polymer Conc. (mg/ml)
dextran
Gen 3.5
Gen 1.5
Gen 7.5
Gen 2.5
poly-L-lysine

FIG. 8

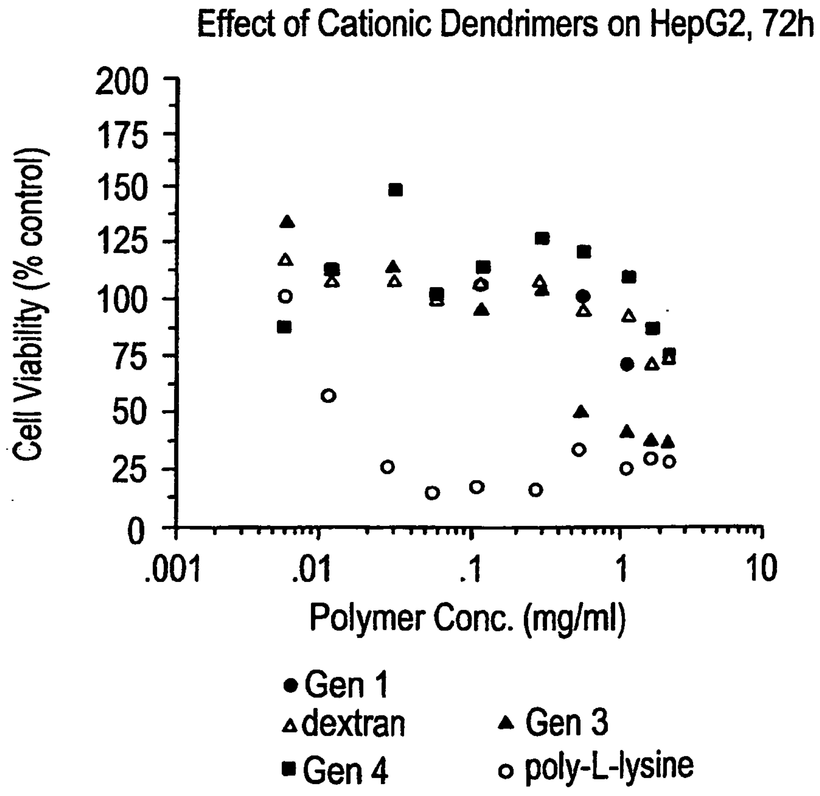
Effect of Cationic Dendrimers on HepG2, 72h
200
175
150
125
100
75
50
25
0
Cell Viability (% control)
.001
.01
.1
1
10
Polymer Conc. (mg/ml)
Gen 1
dextran
Gen 3
Gen 4
poly-L-lysine

FIG. 9

Release of cisplatin at two physiological pH's from dendrimer-platinate, 72h 37°C.

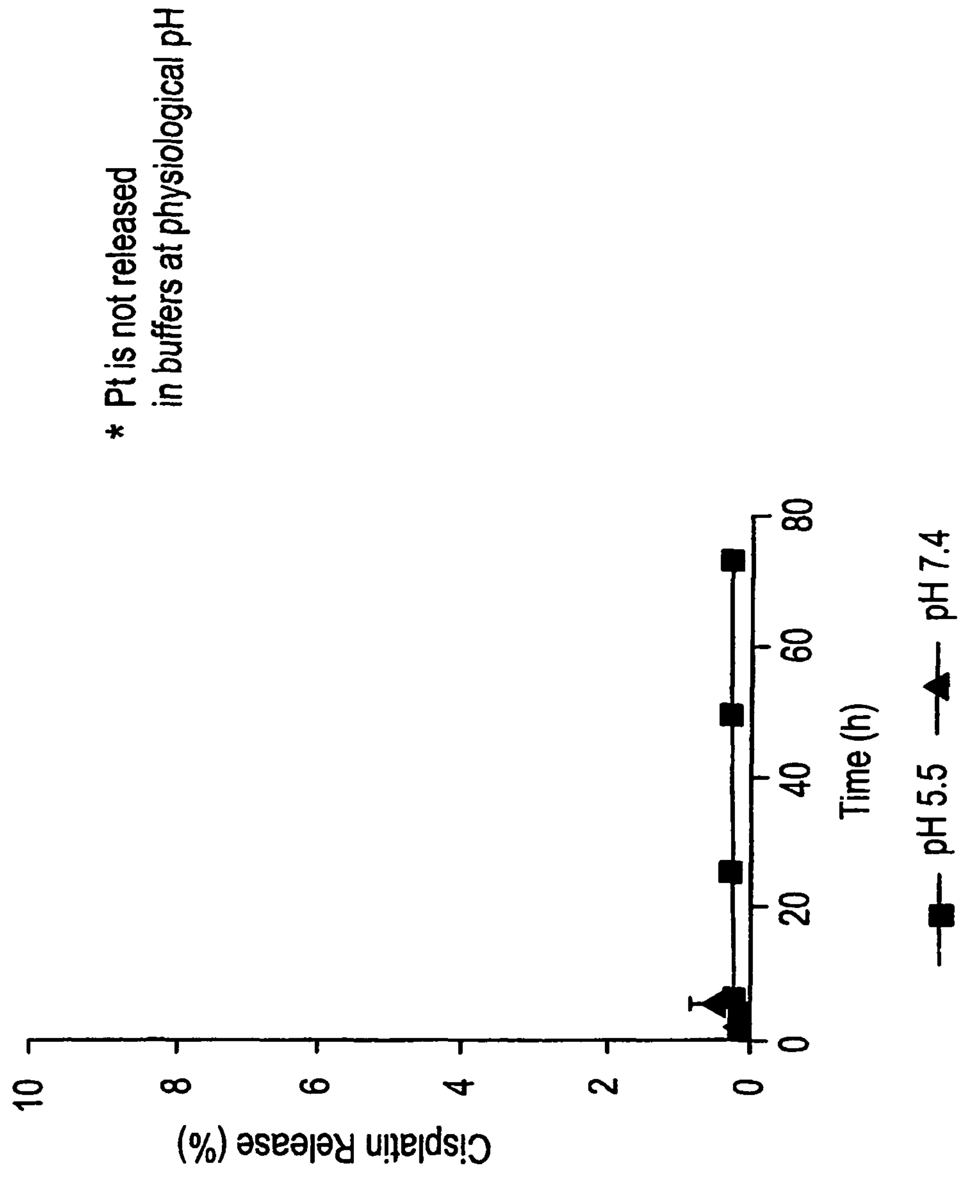

# FIG. 10

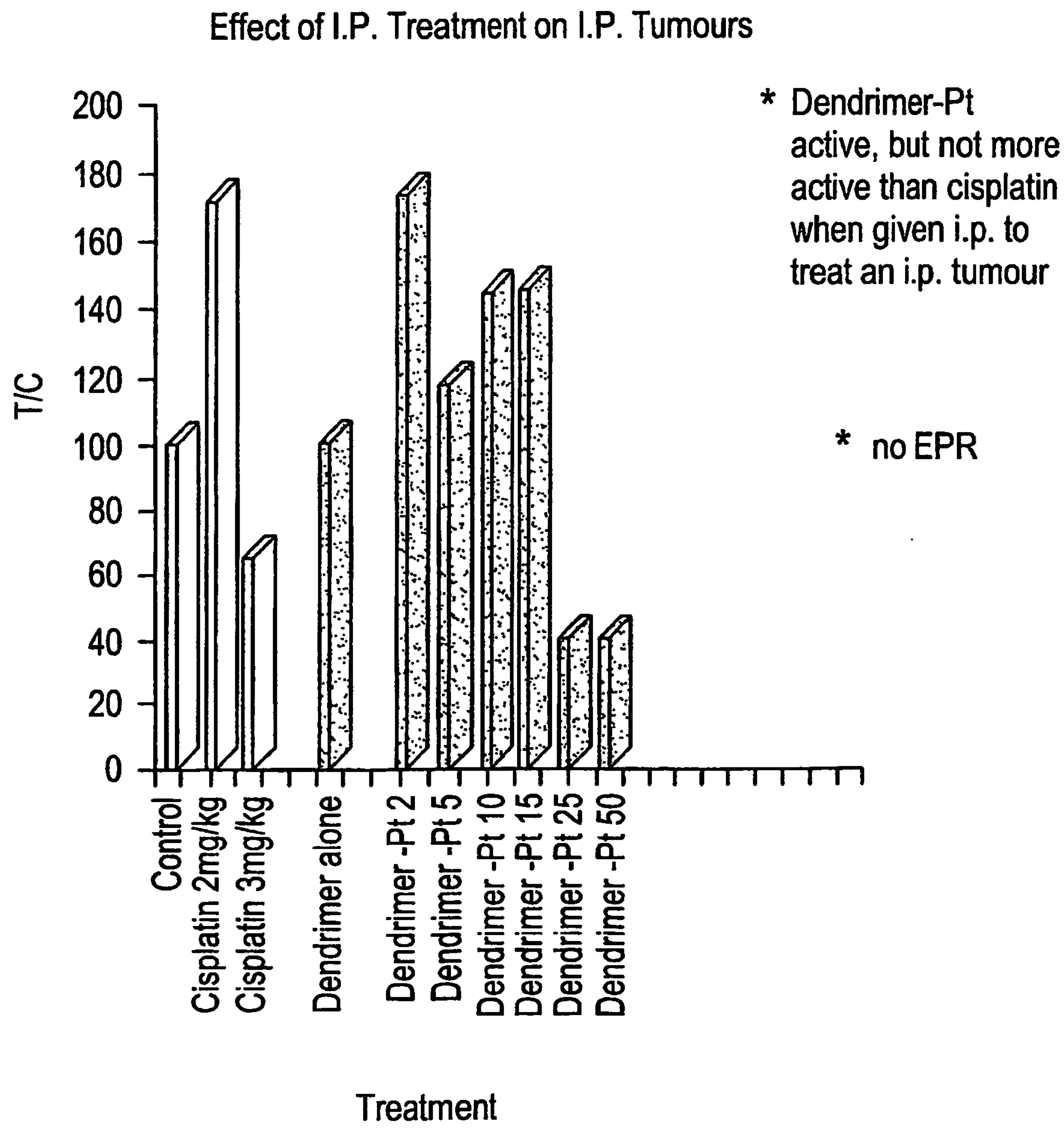
Effect of I.P. Treatment on I.P. Tumours
T/C
200
180
160
140
120
100
80
60
40
20
0
Control
Cisplatin 2mg/kg
Cisplatin 3mg/kg
Dendrimer alone
Dendrimer -Pt 2
Dendrimer -Pt 5
Dendrimer -Pt 10
Dendrimer -Pt 15
Dendrimer -Pt 25
Dendrimer -Pt 50
Treatment
* Dendrimer-Pt active, but not more active than cisplatin when given i.p. to treat an i.p. tumour
* no EPR

FIG. 11

Effect of Dendrimer-Pt on Established
B16 melanoma

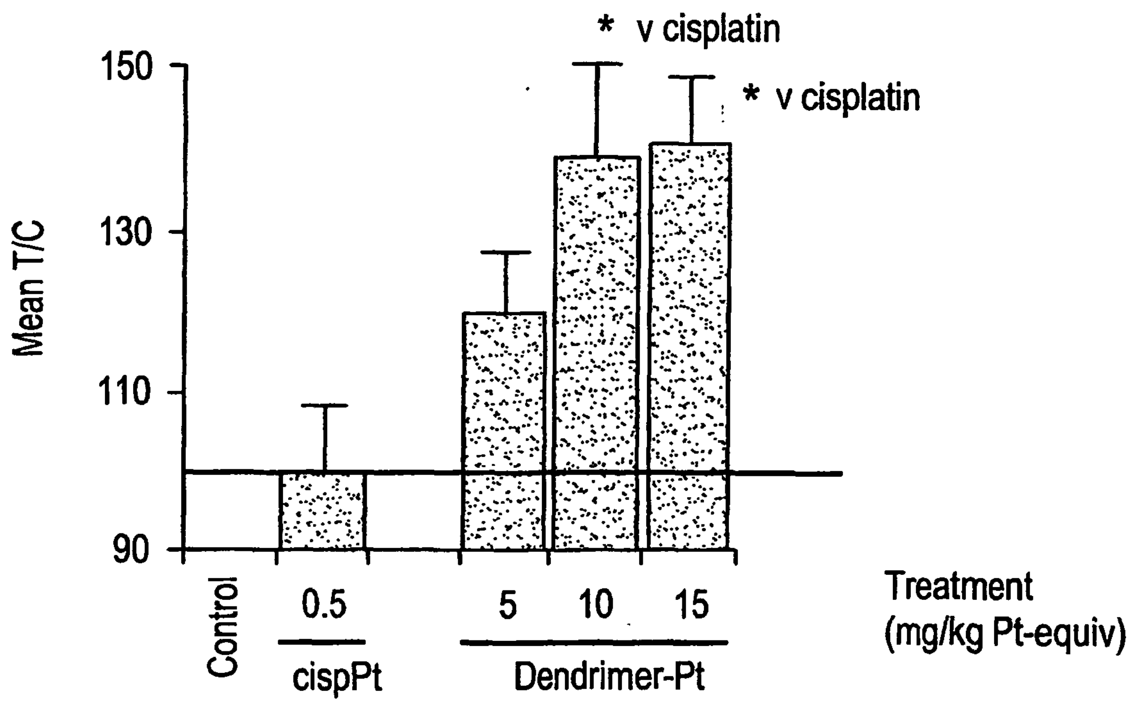

FIG. 12

Accumulation of dendrimer-plantinum and platinum injected i.v. in C57 mice bearing B16F10 s.c. tumour (by AAS)